(19) Europäisches Patentamt
European Patent Office
Office européen des brevets

(11) Publication number: **0 179 864**
**B1**

(12) **EUROPEAN PATENT SPECIFICATION**

(45) Date of publication of patent specification: **22.08.90**

(51) Int. Cl.[5]: **C 12 P 13/00,** C 12 N 1/20

(21) Application number: **85902288.1**

(22) Date of filing: **17.04.85**

(86) International application number:
**PCT/US85/00674**

(87) International publication number:
**WO 85/04900 07.11.85 Gazette 85/24**

# (54) PROCESS FOR PREPARING L-CARNITINE.

(30) Priority: **20.04.84 US 602289**

(43) Date of publication of application:
**07.05.86 Bulletin 86/19**

(45) Publication of the grant of the patent:
**22.08.90 Bulletin 90/34**

(84) Designated Contracting States:
**AT BE CH DE FR GB IT LI LU NL SE**

(56) References cited:
**US-A-3 600 279**
**US-A-3 912 592**
**US-A-4 221 869**
**US-A-4 371 618**

**"Verwertung von Trimethylammoniumverbindugen duch Acinetobacter ------------ calcoaceticus" Archives of Microbiology Vol.112 201-206 1977 ------------- Kleber et al**

(73) Proprietor: **WISCONSIN ALUMNI RESEARCH FOUNDATION**
**614 North Walnut Street**
**Madison, WI 53705 (US)**

(72) Inventor: **SIH, Charles, J.**
**6322 Landfall Drive**
**Madison, WI 53705 (US)**

(74) Representative: **Ellis-Jones, Patrick George Armine et al**
**J.A. KEMP & CO. 14 South Square Gray's Inn**
**London WC1R 5EU (GB)**

(56) References cited:
**"Quantitative Aspects of -butyrobetaine and D-and L-carnitine utili-zation by Growing Cell Cultures of Acinetobacter calcoaceticus and Pseudomonas ------------------------------------------ putide. FEMS Microbiology Letters Vol. 18, pp. 113-116 1983. Miura-Fraboni ------ "Carnitine" Vit. Horm. Vol. 16 pp.57-118 1957 Frankel et al.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for preparing *L*-carnitine.
More specifically, this invention relates to a process for preparing *L*-carnitine by fermentation means.
Still more specifically, this invention relates to a process for resolving *DL*-carnitine microbiologically.
This invention also relates to novel microorganisms which can be used to resolve *DL*-carnitine.

Background Art

It is well documented that a variety of microorganisms are capable of utilizing *L*-carnitine as a sole carbon source. For example, it was reported that a bacterium (tentatively identified as a Pseudomonad), isolated from a polluted stream bed, utilized *L*-carnitine from a *DL*-mixture to produce *D*-carnitine in high optical yield [G. Fraenkel and S. Friedman, *Vitam. Horm.* (N.Y.) 1957 *16*, 73]. Subsequently, the metabolism of *D*- and *L*-carnitine by resting cells of *Pseudomonas putida* and *Acinetobacter calcoaceticus* was investigated. Cells of *Ps. putida* grown on *DL*-carnitine degraded only *L*-carnitine with stoichiometric accumulation of glycine betain [J. Miura-Fraboni and S. England, *FEMS Microbiology Lett. 18* (1983) 113]. Some apparent controversy exists relative to the metabolism of carnitine by *A. calcoaceticus*. One report stated that this organism grew only on *L*-carnitine with the formation of trimethylamine [H. P. Kleber *et al., Arch. Microbiol. 112* (1977) 201]. *D*-carnitine was metabolized, if an additional carbon source, like *L*-carnitine, was present in the incubation mixture, or if the bacteria were preincubated with *L*- or *DL*-carnitine, but no growth was observed on *D*-carnitine as the sole carbon source. On the other hand, J. Miura-Fraboni and S. England claimed that *A. calcoaceticus* utilized for growth both the *D*- and *L*-isomers of carnitine as sole carbon source with stoichiometric formation of trimethylamine, but, until the present invention no microorganisms have been reported which, in an incubation mixture containing *DL*-carnitine, preferentially degrade the unnatural form of carnitine, *D*-carnitine, resulting in the accumulation of the desired natural *L*-carnitine in the medium.

Disclosure of the Invention

It has now been found that a racemic mixture of *DL*-carnitine can be resolved by subjecting it to the fermentative action of enzymes elaborated by microorganisms which are characterized by their unique ability to preferentially metabolize the unnatural *D*-form of the compound, thereby permitting the desired, natural, *L*-carnitine to accumulate in the reaction medium from which the *L*-carnitine can be readily recovered. This process can be represented schematically as follows:

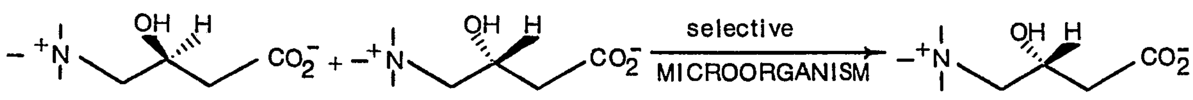


*D* *L* *L*

A wild strain of *A. calcoaceticus* which has the unique characteristic referred to above has been isolated from sewage. Whereas known strains of microorganisms that will metabolize carnitine will not function to resolve *DL*-carnitine since they metabolize *D*- and *L*-carnitine at equal rates, this wild strain which has the characteristics of ATCC 39648, is characterized by its ability to metabolize *D*-carnitine at a faster rate than *L*-carnitine in liquid or solid media. Thus, this strain responds to the criteria required of a microorganism for purposes of the process of the present invention and has the unique ability to resolve *DL*-carnitine and permit recovery of the natural and desired form of carnitine, *L*-carnitine.

In addition to the above identified wild strain, a mutant of that strain, which has the characteristics of ATCC 39647, has been prepared. This mutant is distinguished from the wild strain by its ability to permit the accumulation of greater quantities of *L*-carnitine in the reaction medium when utilized to resolve *DL*-carnitine through fermentative action.

The mutant, ATCC 39647, can be obtained by growing *A. calcoaceticus* ATCC 39648 on a suitable medium containing *DL*-carnitine and then subjecting to nitrosoguanidine (NTG) mutagenesis. The mutant is then selected on agar plates containing either *D*- or *L*-carnitine as sole carbon source. The desired mutant *A. calcoaceticus* ATCC 39647 will grow poorly on a medium containing *L*-carnitine as the sole carbon source but will grow rapidly on plates containing *D*-carnitine as sole carbon source. This selection was achieved by the replica plating method [D. F. Spooner and G. Sykes in *Methods in Microbiology*, Vo. 7B, p. 244 (1972)]. It will be evident to those skilled in the art that other mutation procedures may be successfully utilized to produce mutants suitable for the process of this invention. Thus, instead of using nitrosoguanidine to achieve mutagenesis other chemical mutagens can be employed such as, for example, sodium nitrite, ethyleneimine, 8-azaguanine, N-methyl N'-nitrosoguanine or nitrogen mustards followed by selection. In addition, physical mutagenesis may be employed utilizing UV light and ionizing or high energy radiation such as X-rays, gamma rays and electron beams. Mutagenesis may also be accomplished through presently available molecular biology techniques, e.g. transduction, transformation, conjugation, cell fusion, recombinant DNA techniques can be used to engineer new microorganism strains.

Accordingly the present invention provides a process for preparing *L*-carnitine or a salt thereof

characterised by subjecting a racemic mixture of *DL*-carnitine or a salt thereof to the fermentative action of enzymes elaborated by a microorganism which is characterised by its unique ability to degrade preferentially the unnatural form, *D*-carnitine, in said mixture and permit the desired natural form, *L*-carnitine, to accumulate.

The preferred process of the present invention comprises cultivating a microorganism having the identifying characteristics of *A. calcoaceticus* ATCC 39647 in an aqueous nutrient medium under aerobic conditions in a mineral salts medium containing *DL*-carnitine as the sole carbon source. Alternatively, the *DL*-carnitine can be added to the microorganism culture after it has been grown on a nutrient medium.

The preferred concentration of the *DL*-carnitine (as hydrochloride) is about 0.1 to about 100 grams per liter, depending upon the ratio of *DL*-carnitine and the ammonia concentration of the medium. It will be understood that with increasing *DL*-carnitine chloride concentrations, more ammonium ion is required to achieve the optimum rate of metabolism of the *D*-isomer. It will also be understood that the terms *DL*-carnitine and *DL*-carnitine chloride are used interchangeably throughout this specification since they can be used interchangeably in the process of the invention unless otherwise indicated. The same interchangeability is also used with respect to *D*- and *L*-carnitine and their corresponding chlorides.

The duration of the process is generally from 24 hours to 5 days or more. The incubation temperature is usually from 25° to 37°C. The contents are suitably aerated with sterilized air and agitated to facilitate growth of the microorganism and thus enhance the effectiveness of the process.

Upon completion of the fermentation process, the desired *L*-carnitine can be recovered by means well known in the art. For example, the fermentation liquor and cells can be first separated by conventional methods, e.g., filtration or centrifugation after which the fermentation liquor may be vacuum distilled to dryness. The resulting residue containing the desired *L*-carnitine may be extracted with anhydrous ethanol, the ethanolic extract evaporated to dryness *in vacuo*, and the *L*-carnitine converted into its hydrochloride salt by treating the residue with 6N HCl. After evaporation of the aqueous solution to dryness, *L*-carnitine chloride may be crystallized from a mixture of absolute ethanol-acetone. Alternatively *L*-carnitine inner salt may be obtained by chromatographing the residue of the ethanolic extract on a Dowex-1-OH ion exchange column [F. Strack and I. Lorenz, *Z. Physiol. Chemie 318* (1960) 129].

It will be appreciated that, if desired, the cells of the microorganism may be immobilized [S. Fukui, A. Tanaka, Ann. Rev. of Microbiol. 1982, Vol. 36, p. 145] to permit the process to be carried out in a continuous manner. It will also be evident that to enhance stereo-selectivity the growth medium may be modified or further mutagenesis of the mutant accomplished.

The following examples are to be considered illustrative only of the present invention and are not to be construed as limiting. Percentages are given by weight and solvent mixture proportions are expressed by volume unless otherwise stated. The isolated *L*-carnitine was characterized by proton magnetic resonance spectra, melting point, mobility on thin layer chromatography and optical rotation.

Example 1

Isolation of the Wild Type (df-2) of *Acinetobacter calcoaceticus* ATCC 39648

*A. calcoaceticus* ATCC 39648 was isolated from a sewerage sample obtained from a skimming tank using a selection medium via the following procedure:

Ten milliliters of greasy surface water from a sewerage skim tank (Nine Springs treatment plant, Madison, WI) was collected and 1 ml of this sample was added to a 250 ml Erlenmeyer flask containing 50 ml of modified Johnson's medium (Medium A):

Medium A

| | |
|---|---|
| Yeast extract (Difco) | 50 mg |
| $KH_2PO_4$ | 5.5 g |
| $Na_2HPO_4$ | 10.0 g |
| $(NH_4)_2HPO_4$ | 2.0 g |
| $NH_4H_2PO_4$ | 1.5 g |
| $CaCl_2$ | 15 mg |
| $MgDO_4 \cdot 7H_2O$ | 200 mg |
| $Fe_2(SO_4)_3$ | 0.6 mg |
| $ZnSO_4 \cdot 7H_2O$ | 0.2 mg |
| $CuSO_4 \cdot 5H_2O$ | 0.2 mg |
| $MnSO_4 \cdot H_2O$ | 0.2 mg |
| Deionized distilled water | 1 liter |

*DL*-carnitine HCl (Sigma) (10 g/l) is added and the pH is adjusted to 6.8 with 4N NaOH prior to sterilization at 121°C for 20 minutes. The inoculated flask was incubated on a rotary shaker (250 rpm, 2" stroke, 27°C). After 16 hours, one ml of the turbid broth was transferred to another flask containing like medium and reincubated on a rotary shaker for 16 hours. The procedure was repeated for a third time after which the turbid broth was diluted serially using M/15 phosphate buffer, pH 7.4. An aliquot (0.1 ml) of each dilution was spread evenly over the agar surface of a petri plate containing 1% of *DL*-carnitine chloride and 2% agar in modified Johnson's medium A. Different colony types were selected from plates that contained between 30 and 300 colonies per plate. The colonies were streaked on 1% *DL*-carnitine chloride modified Johnson's medium agar plates and checked for purity. The pure isolates were then tested for their ability to utilize *D*-carnitine and *L*-carnitine as a sole carbon source on modified Johnson's medium containing 0.5% of either *D*- or *L*-carnitine. It was observed that the isolate "df-2" grew considerably faster on *D*-carnitine than on *L*-carnitine. Hence this wild strain was chosen for the subsequent mutagenesis study.

Example 2
Preparation of Mutant *A. calcoaceticus* ATCC 39647
from Wild type "df-2", *A. calcoaceticus* ATCC 39648

a) Nitrosoguanidine Mutagenesis

The wild type "df-2", identified as *Acinetobacter calcoaceticus* (University Hospital Clinical Microbiology Laboratory, Madison, WI), was maintained on 1% *DL*-carnitine chloride modified Johnson's agar medium slants and stored at 4°C. A 250 ml Erlenmeyer flask containing 50 ml of 1% *DL*-carnitine chloride modified Johnson's medium was inoculated from a slant and then incubated for 16 hours on a rotary shaker (250 rpm, 2" stroke, 27°C). To this overnight growth was added N-ethyl-N'-nitro-N-nitrosoguanidine (Aldrich) (NTG) to a final concentration of 300 μg/ml of medium. The flask was placed back on the rotary shaker and incubated for exactly 30 minutes. It had been previously established that 300 μg NTG/ml medium for 30 minutes killed approximately 99.9% of viable cells. After 30 minutes exposure to NTG, the cells were centrifuged down (10,000 rpm, 15 minutes). The cell pellet was resuspended in 5 ml of modified Johnson's medium containing 1% *DL*-carnitine chloride. Two ml of this suspension was used to inoculate a 250 Erlenmeyer flask containing 50 ml of 1% *DL*-carnitine chloride modified Johnson's medium. After the flask was incubated on a rotary shaker (200 rpm, 27°C) for 16 hours, the turbidity was determined on a Gilford UV-visible spectrophotometer (Model 240) by monitoring the absorbance at 600 nm. This data allowed the calculation of the proper dilution to be used for plating and mutant selection.

b) Selection and isolation of mutant *A. calcoaceticus* ATCC 39647

Mutagenized cells, as described above, were serially diluted in M/15 phosphate buffer pH 7.4 and spread onto plates containing 1% *DL*-carnitine chloride modified Johnson's medium. After incubation at 28°C for about four days, the resulting colonies were replicated on modified Johnson's medium A containing 0.5% *L*-carnitine or 0.5% *D*-carnitine.

Colonies which grew well on the *D*-carnitine plate but poorly on the *L*-carnitine plate were purified by streaking onto 0.5% *D*-carnitine modified Johnson's agar plates (medium A). After growth at 28°C, individual colonies were picked from the agar plates onto slants and then evaluated in shake flasks.

c) Shake flask evaluation

Shake flasks (250 ml) containing 50 ml of 1% *DL*-carnitine chloride modified Johnson's liquid medium A were inoculated with several loops of cells taken from an agar slant. After 24 hours on a rotary shaker, 50 ml of this turbid broth served as the inoculum for another 2 L Erlenmeyer flask containing 500 ml of the same medium. After incubation at 27°C on rotary shaker (250 rpm, 2" stroke) for 44 hours, 100 ml of the flask contents were evaporated to dryness *in vacuo* and the residue was extracted with absolute ethanol. The ethanolic extract was again evaporated to dryness *in vacuo* and the residue was dissolved in 10 ml of 6N HCl to convert carnitine into its chloride salt. The aqueous sample was then concentrated to dryness *in vacuo* and the residual *L*-carnitine chloride was crystallized from absolute ethanol-acetone. The high negative rotation of the product ($[\alpha]_D^{25}$ −23.07°C ((c, 3.5 $H_2O$)) confirms the selective degradation of *D*-carnitine by the novel mutant produced from the parent wild strain *A. calcoaceticus* ATCC 39648.

*Acinetobacter Calcoaceticus* and the strains identified by ATCC 39647 and ATCC 39648 have the following properties:

Morphological Characteristics

Medium — Difco nutrient agar (Difco Laboratories, Detroit, Michigan);

Shape and size of cell — Rods 1—1.6 μm in diameter and approximately 1.5—22 μm in length;

becoming spherical (coccoid rods) in the stationary phase of growth;

often occur in pairs and in chains of variable length.

No flagella.

No spores.

Gram negative.

Non acid fast.

Culture Growth Condition

Medium — Difco nutrient agar (Difco Laboratories, Detroit, Michigan)

Colonies are relatively large (2—3 mm in 24 hrs.) are opaque and nonpigmented;

Organism can grow in a simple mineral salts medium containing a single carbon energy source such as ethanol, acetate, lactate, pyruvate, malate or alpha-ketoglutarate;

Ammonium salts serve as nitrogen sources;

Gelatin not liquefied in gelatine stab culture and growth limited to top 3 mm of medium;

Alkaline reaction in litmus milk with no coagulation or liquefaction and very poor growth.

Physiological Properties

Nitrate reduction — negative

Denitrification reaction — negative

MR test — negative

Indole production — negative

VP test — negative

Hydrogen sulfide production — very, very weak

Starch hydrolysis — negative

Utilization of nitrate — positive

Utilizes ammonium salts

Pigment Production — yellowish intracellular water soluble pigment which is released into medium upon autolysis after 2—3 days.

Urease — positive

Oxidase — positive

Growth conditions — pH 5—8.5

— temperature 5°—35°C

— will not grow anaerobically in any medium.

Production of acids and gas in following:

| | | ATCC 39648 | | ATCC 39647 | |
|---|---|---|---|---|---|
| | | Open | Covered | Open | Covered |
| 1) | L-arabinose | A | — | A | — |
| 2) | D-xylose | A | — | A | — |
| 3) | D-glucose | A | A$^{\mu}$ | A | — |
| 4) | D-mannose | A | — | A | — |
| 5) | D-fructose | A$^{\mu}$ | — | A$^{\mu}$ | — |
| 6) | D-galactose | A | — | A | — |
| 7) | maltose | — | — | — | — |
| 8) | sucrose | — | — | — | — |
| 9) | lactose | — | — | — | — |
| 10) | trehalose | — | — | — | — |
| 11) | D-sorbital | — | — | — | — |
| 12) | D-mannitol | — | — | — | — |
| 13) | inositol | — | — | — | — |
| 14) | glycerine | — | — | — | — |
| 15) | starch | — | — | — | — |

Key: A = acid reaction
μ = weak reaction
— = no change or alkaline reaction

Other Characteristics
- Oxidizes ethyl alcohol;
- Has high tolerance for sodium chloride (2—3% NaCl);
- Can grow at 33°C
- Phenylalanine is not deaminated;
- Lysine is not decarboxylated;
- Ornithine is not decarboxylated.

The strains identified by the depository numbers ATCC 39647 and 39648 were deposited with the American Type Culture Collection, 12301 Parklawn Drive, Rockville, Maryland 20852—1776 on April 2, 1984.

Example 3
Preferential Utilization of *D*-carnitine by
*A. calcoaceticus* ATCC 39648

The surface growth from a one week old slant of *A. calcoaceticus* ATCC 39648 was suspended in 5 ml of saline (0.85%) solution. Two ml of this suspension were used to inoculate 50 ml of the modified Johnson's medium A containing 1% *DL*-carnitine chloride above held in a 250 ml Erlenmeyer flask (f-1 stage). The flask was incubated at 25°C on a rotary shaker (250 rpm/min — 2 inch radius) for 24 hours, after which a 10% by volume transfer was made to a two liter Erlenmeyer flask (F-2 stage), containing 500 ml of the same medium. After 12 hours on the rotary shaker, 100 ml of the medium was removed centrifuged, and then evaporated to dryness *in vacuo*. The residue was extracted with hot ethanol and the ethanolic extract was concentrated to dryness *in vacuo*. The residue was dissolved in 10 ml of 6N HCl and the aqueous solution was concentrated to dryness. Crystallization of the residue from absolute ethanol-acetone afforded a sample of carnitine chloride (216 mg, 43% yield) ($[\alpha]_D^{25}$ −12.0°) (c, 7.2 $H_2O$) indicating an enantiomeric excess (*ee*) of 0.51.

Example 4

The surface growth from a one week old slant of *A. calcoaceticus* ATCC 39648 was suspended in 5 ml of medium A. Four ml of this suspension was used to inoculate 50 ml of the modified Johnson's medium A containing 1% *DL*-carnitine chloride above held in a 250 ml Erlenmeyer flask (F-1 stage). The flask was incubated at 25°C on a rotary shaker (250 rpm/min — 2 inch radius) for 24 hours, after which a 10% by volume transfer was made to a two liter Erlenmeyer flask (F-2 stage) containing 500 ml of the following medium (Medium B):

Medium B

| | |
|---|---|
| Yeast extract (Difco) | 50 mg |
| $KH_2PO_4$ | 5.5 g |
| $Na_24PO_4$ | 10.0 g |
| $(NH_4)_2HPO_4$ | 4.0 g |
| $NH_4H_2PO_4$ | 3.5 g |
| $CaCl_2$ | 15 mg |
| $MgSO_4 \cdot 7H_2O$ | 200 mg |
| $Fe_2(SO_4)_3$ | 0.6 mg |
| $ZnSO_4 \cdot 7H_2O$ | 0.2 mg |
| $CuSO_4 \cdot 5H_2O$ | 0.2 mg |
| $MnSO_4 \cdot H_2O$ | 0.2 mg |
| Deionized distilled water | 1 liter |

*DL*-carnitine HCl (Sigma) (20 g/l) is added and the pH is adjusted to 6.8 with 4N NaOH prior to sterilization at 121°C for 20 minutes.

After incubation for 44 hours on the rotary shaker at 25°C, 50 ml of the contents were centrifuged to remove the cells and the supernatant was then evaporated to dryness. *L*-carnitine chloride (196 mg, 38% yield) was isolated following the procedure described in Example 3. The optical purity was estimated to be greater than 96.5% *ee* from the $[\alpha]_D^{25}$ −22.9° (c, 2.65 $H_2O$) value.

Example 5

The procedure of Example 4 was followed except that the concentration of *DL*-carnitine chloride for the F-2 stage was 50 g/l in a 250 ml Erlenmeyer flask containing 50 ml of the same medium. After the F-2 stage was allowed to proceed for 68 hours, *L*-carnitine chloride (180 mg, 42% yield) was isolated via the same procedure (Example 3) from 17 ml of the fermentation broth. The optical purity of the sample was greater than 86% *ee* as evidenced by the $[\alpha]_D^{25}$ −20.43° (c, 2.36 $H_2O$) value.

Example 6

The procedure of Example 2c was followed except the 1% *DL*-carnitine chloride concentration consisted of a mixture of 0.3% *L*-carnitine chloride and 0.7% *D*-carnitine chloride in medium A. The fermentation was carried out in a 2 liter Erlenmeyer flask containing 250 ml of medium A using *A. calcoaceticus* ATCC 39647. After the F-2 stage was allowed to proceed for 44 hours, 100 ml of the broth was removed and the *L*-carnitine chloride isolated [36 mg (12% yield), $[\alpha]_D^{25}$ −21.2°, c, 3.6, $H_2O$].

Example 7

The procedure of Example 2c was followed except the 2% *DL*-carnitine chloride concentration consisted of a mixture of 1.6% *L*-carnitine chloride and 0.4% *D*-carnitine chloride in medium A using *A. calcoaceticus* ATCC 39647. After the F-2 stage was allowed to proceed for 44 hours, 100 ml of the broth was removed and the *L*-carnitine chloride isolated [551 mg (34% yield), $[\alpha]_D^{25}$ −23.05°, c, 2.6, $H_2O$].

Example 8

The procedure of Example 2c was followed except the concentration of *DL*-carnitine chloride for the F-2 stage was 50 g/l, which consisted of 3 parts of *L*-carnitine chloride and 7 parts *D*-carnitine chloride in 250 ml

of medium B held in a two liter Erlenmeyer flask. After incubation with *A. calcoaceticus* ATCC 39647 for 69 hours (F-2 stage), 50 ml of the broth was centrifuged (9000 × 6 for 20 minutes) to remove the cells. The supernatant was evaporated to dryness *in vacuo* and the residue was extracted with anhydrous ethanol. The ethanolic extract was concentrated to dryness and the residue was dissolved in distilled water and chromatographed over a Dowex 1—X—4 OH column (5 × 22 cm) to obtain *L*-carnitine [140 mg (23% yield), $[\alpha]_D^{25}$ −29.2°, c, 1.5 $H_2O$].

Example 9

The procedure of Example 8 was followed except that the concentration of *DL*-carnitine chloride for the F-2 stage was 50 g/l, which consisted of 8 parts of *L*-carnitine chloride and 2 parts of *D*-carnitine chloride. *L*-carnitine inner salt was isolated as before from 50 ml of the fermentation mixture (F-2 stage, 69 hours) [1.216 g (75% yield), $[\alpha]_D^{25}$ −30.4°, c = 2.8, $H_2O$].

Example 10

The procedure of Example 8 was followed except that the concentration of *DL*-carnitine chloride for the F-2 stage was 50 g/l. After the F-2 stage was allowed to proceed for 90 hours, 40 ml of the fermentation broth was removed and centrifuged. *L*-carnitine inner salt was isolated as before [0.62 g (75% yield), $[\alpha]_D^{25}$ −27.0°, c = 4.4, $H_2O$].

**Claims**

1. A process for preparing *L*-carnitine or a salt thereof characterised by subjecting a racemic mixture of *DL*-carnitine or a salt thereof to the fermentative action of enzymes elaborated by a microorganism which is characterised by its unique ability to degrade preferentially the unnatural form, *D*-carnitine, in said mixture and permit the desired natural form, *L*-carnitine, to accumulate.

2. A process according to Claim 1 wherein the bacterium has the characteristics of *Acinetobacter calcoaceticus* ATCC 39647, or ATCC 39648 or a mutant thereof.

3. A process according to Claim 1 or 2 which is carried out in a growing culture of the microorganism in an aqueous nutrient medium under aerobic conditions.

4. A process according to Claim 3 which is carried out in a medium comprising mineral salts, mineral ions and *DL*-carnitine as the sole carbon source and the rate of metabolism of the *D*-isomer of carnitine is increased by increasing the ammonium ion concentration in the reaction medium as the *DL*-carnitine concentration increases.

5. A process according to any one of the preceding claims for resolving *DL*-carnitine, absent the prerequisite of preliminarily preparing derivatives thereof, which comprises subjecting *DL*-carnitine per se to the fermentative action of enzymes elaborated by the said microorganism to preferentially degrade the unnatural, *D*-form and recovering the *L*-form from the reaction medium.

6. Bacterium of the family *Neisseriaceae* characterised by their ability to resolve a racemic mixture of *DL*-carnitine by degrading preferentially the unnatural, *D*-form of carnitine in the racemic mixture and permitting the *L*-form to accumulate.

7. A microorganism of the strain *Acinetobacter calcoaceticus*, having the identifying characteristics of ATCC 39648.

8. A microorganism of the strain *Acinetobacter calcoaceticus*, having the identifying characteristics of ATCC 39647.

9. A culture containing the microorganism *Acinetobacter calcoaceticus*, said culture being capable of resolving a racemic mixture of *DL*-carnitine by preferentially degrading the unnatural D-form in the racemic mixture.

**Patentansprüche**

1. Verfahren zur Herstellung von *L*-Karnitin oder einem Salz hiervon, dadurch gekennzeichnet, daß eine razemische Mischung von *DL*-Karnitin oder einem Salze hiervon fermentativer Einwirkung von Enzymen unterworfen wird, wie sie von einem Mikroorganismus ausgeübt wird, der durch seine besondere Fähigkeit charakterisiert ist, bevorzugt die unnatürliche Form, *D*-Karnitin, in dieser Mischung abzubauen und die Anreicherung der gewünschten natürlichen Form *L*-Karnitin, gestattet.

2. Verfahren nach Anspruch 1, wobei das Bakterium die charakteristischen Merkmale von *Acinetobacter calcoaceticus* ATCC 39647 oder ATCC 39648 oder einem Mutanten hiervon aufweist.

3. Verfahren nach Anspruch 1 oder 2, das in einer Wachstumskultur des Mikroorganismus in einem wässrigen Nährmedium unter aeroben Bedingungen durchgeführt wird.

4. Verfahren nach Anspruch 3, das in einem Medium durchgeführt wird, das mineralische Salze, mineralische Ionen und *DL*-Karnitin als einzige Kohlenstoffquelle enthält und bei dem die Geschwindigkeit des Metabolismus des *D*-Isomeren des Karnitins erhöht wird indem die Ammoniumionen-Konzentration in dem Reaktionsmedium entsprechend dem Anstieg der *DL*-Konzentration gesteigert wird.

5. Verfahren nach einem der vorhergehenden Ansprüche zum Zwecke des Aufschlusses von *DL*-Karnitin ohne daß zunächst Derivate hiervon hergestellt werden mußten indem *DL*-Karnitin als solches

fermentativer Einwirkung von Enzymen unterworfen wird wie sie von dem erwähnten Mikroorganismus ausgeübt wird, um bevorzugt die unnatürliche *D*-Form abzubauen, und die *L*-Form aus dem Reaktionsmedium gewonnen wird.

6. Bakterium der Familie *Neisseriaceae* gekennzeichnet durch ihre Fähigkeit eine razemische Mischung von *DL*-Karnitin aufzuschließen durch bevorzugten Abbau der unnatürlichen, *D*-Form des Karnitins in der razemischen Mischung und Gestattung einer Anreicherung der *L*-Form.

7. Mikroorganismus von dem Stamm *Acinetobacter calcoaceticus* mit den Identifizierungsmerkmalen von ATCC 39648.

8. Mikroorganismus von dem Stamm *Acinetobacter calcoaceticus* mit den Identifizierungsmerkmalen von ATCC 39647.

9. Eine Kultur enthaltend den Mikroorganismus *Acinetobacter calcoaceticus*, wobei die Kultur in der Lage ist eine razemische Mischung von *DL*-Karnitin aufzuschließen indem die unnatürliche *D*-Form in der razemischen Mischung bevorzugt abgebaut wird.

**Revendications**

1. Procédé de préparation de la *L*-carnitine ou de l'un de ses sels, caractérisé en ce que l'on soumet un mélange racémique de *DL*-carnitine ou un sel dudit mélange à l'action de fermentation d'enzymes élaborés par un micro-organisme qui se caractérise par son aptitude particulière à dégrader préférentiellement la forme non naturelle *D*-carnitine dans ledit mélange et à permettre l'accumulation de la forme naturelle souhaitée *L*-carnitine.

2. Procédé selon la revendication 1, dans lequel la bactérie a les caractéristiques de *Acinetobacter calcoacéticus* ATCC 39647, ou ATCC 39648 ou d'un mutant de ces derniers.

3. Procédé selon la revendication 1 ou 2, qui est effectué dans une culture de croissance du microorganisme dans un milieu nutritif aqueux, dans des conditions aérobies.

4. Procédé selon la revendication 3, qui est effectué dans un milieu comprenant des sels minéraux, des ions minéraux et de la *DL*-carnitine en tant que seule source de carbone, et dans lequel on accroît la vitesse de métabolisme de l'isomère *D* de la carnitine en augmentant la concentration en ions ammonium dans le milieu réactionnel à mesure que la concentration en *DL*-carnitine augmente.

5. Procédé selon l'une quelconque des revendications précédentes pour dédoubler la *DL*-carnitine, la nécessité préalable d'une préparation préliminaire de dérivés de cette dernière étant absente, procédé qui consiste à soumettre la *DL*-carnitine elle-même à l'action de fermentation d'enzymes élaborés par ledit microorganisme pour dégrader préférentiellement la forme *D* non naturelle et récupérer la forme *L* à partir du milieu réactionnel.

6. Bactérie de la famille *Neisseriaceae* caractérisée par son aptitude à dédoubler un mélange racémique de *DL*-carnitine en dégradant préférentiellement la forme *D* non naturelle de la carnitine dans le mélange racémique et en permettant l'accumulation de la forme *L*.

7. Micro-organisme de la souche *Acinetobacter calcoaceticus*, ayant les caractéristiques d'identification de ATCC 39648.

8. Micro-organisme de la souche *Acinetobacter calcoaceticus*, ayant les caractéristiques d'identification de ATCC 39647.

9. Culture contenant le micro-organisme *Acinetobacter calcoaceticus*, ladite culture étant capable de dédoubler un mélange racémique de *DL*-carnitine en dégradant préférentiellement la forme *D* non naturelle dans le mélange racémique.